Europäisches Patentamt

European Patent Office

Office européen des brevets

(11) Numéro de publication: **0 218 539 B1**

(12) **FASCICULE DE BREVET EUROPEEN**

(19)

(45) Date de publication de fascicule du brevet: **08.04.92**  (51) Int. Cl.⁵: **A61L 2/18, //G02C13/00**

(21) Numéro de dépôt: **86450020.2**

(22) Date de dépôt: **24.09.86**

(54) **Appareil nettoyeur et stérilisateur des lentilles de contact.**

(30) Priorité: **30.09.85 FR 8514571**

(43) Date de publication de la demande:
**15.04.87 Bulletin 87/16**

(45) Mention de la délivrance du brevet:
**08.04.92 Bulletin 92/15**

(84) Etats contractants désignés:
**AT BE CH DE FR GB IT LI LU NL SE**

(56) Documents cités:
**US-A- 4 013 410**
**US-A- 4 143 116**
**US-A- 4 381 285**

(73) Titulaire: **Barrau, Bernard**
**10, Rue Delcasse**
**F-09000 Foix(FR)**

(72) Inventeur: **Barrau, Bernard**
**10 rue Delcasse**
**F-09000 Foix(FR)**
Inventeur: **Bielsa, Francis**
**Goutte Madère**
**F-09000 Pailhes(FR)**

(74) Mandataire: **Ravina, Bernard**
**Cabinet Bernard RAVINA 24, boulevard Riquet**
**F-31000 Toulouse(FR)**

EP 0 218 539 B1

## Description

La présente invention concerne un appareil nettoyeur et stérilisateur d'objets, par exemple des lentilles de contact.

Elle concerne plus particulièrement un appareil qui, à partir d'une durée prédéterminée de séjour des dites lentilles dans une solution stérilisatrice, les transfère dans une solution de neutralisation pour réduire les résidus de la première solution et permettre de stocker convenablement les lentilles en attendant leur utilisation.

Pour stériliser quotidiennement chimiquement par système oxydant les lentilles de contact, on les place, pendant une durée prédéterminée, dans une solution stérilisatrice ( peroxyde d'hydrogène par exemple) .

A la fin de cette période la stérilisation est complète, on les transfère dans une solution de neutralisation : on les laisse tremper pendant au moins un temps préconisé par le fabricant de produits. Passé ce délai le porteur peut mettre ses lentilles qui sont ainsi nettoyées, asceptisées, et conservées en état d'hydratation.

La durée pendant laquelle les lentilles de contact séjournent dans la solution stérilisatrice doit être suffisante pour qu'une stérilisation complète ait lieu. Par contre, une prolongation non nécessaire de la dite durée aide à faire accrocher un nombre, de plus en plus, important de molécules actives sur la surface et en profondeur des dites lentilles. Ceci nécessite une durée plus importante pour neutraliser ces molécules.

Hebdomadairement, pour enlever les dépots de protéines sur les lentilles, on les fait tremper dans une solution contenant des déprotéinisants pendant un temps prédéterminé. Ensuite soit on les transfère dans une solution de rinçage soit on procède au cycle de nettoyage quotidien décrit précédemment.

On connaît le brevet US 4 381 285 dans lequel l'étui des lentilles est éjecté vers le contenant de neutralisation une fois que sa durée de stérilisation est accomplie dans le contenant de stérilisation.

Un tel appareil évite de faire manuellement l'opération de transfert des lentilles de contact de la solution stérilisatrice à la solution de neutralisation au bout d'une durée prédéterminée.

Cependant l'appareil qu'on vient de citer a les désavantages suivants :

- faire subir aux lentilles un choc mécanique lors de l'éjection,
- faire transférer une partie de la solution stérilisatrice ,en même temps que les lentilles,dans la solution de neutralisation vu le façon d'effectuer le dit transfert,
- faire éclabousser les deux solutions , la solution stérilisatrice et la solution de neutralisation, lors du dit transfert.

Pour pallier ces inconvénients, la présente invention propose un appareil pour effectuer un tel transfert sans mouvement brutal et tout en donnant le temps à l'étui de s'égoutter.

A cet effet, la présente invention a pour objet un appareil stérilisateur tel que défini à la revendication 1.

La présente invention sera mieux comprise à la lecture de la description détaillée ci-aprés illustrée par des dessins dans lesquels :

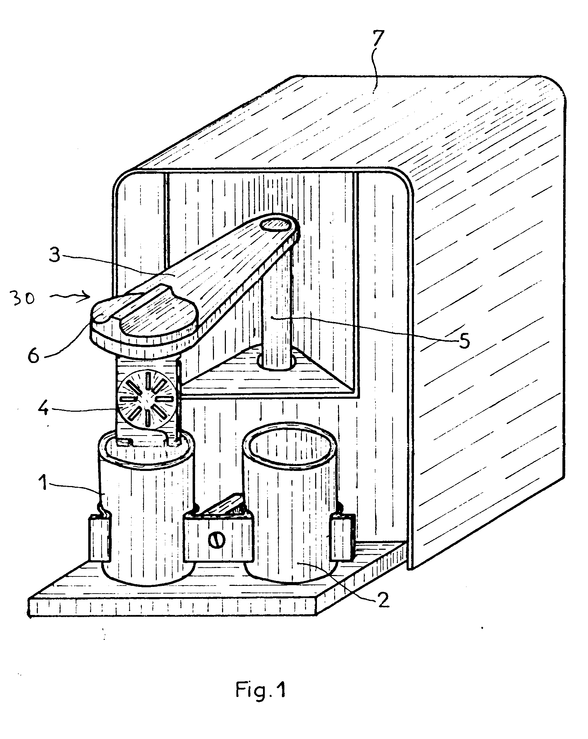

- la figure 1 est une vue en perspective de l'appareil
- la figure 2 est une vue qui montre le mécanisme engendrant le mouvement pour effectuer le dit transfert,

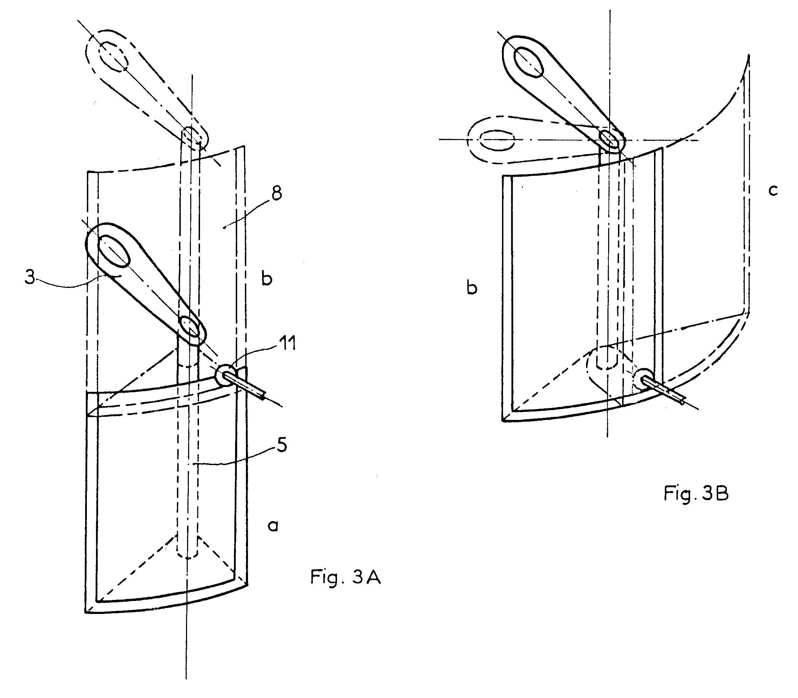

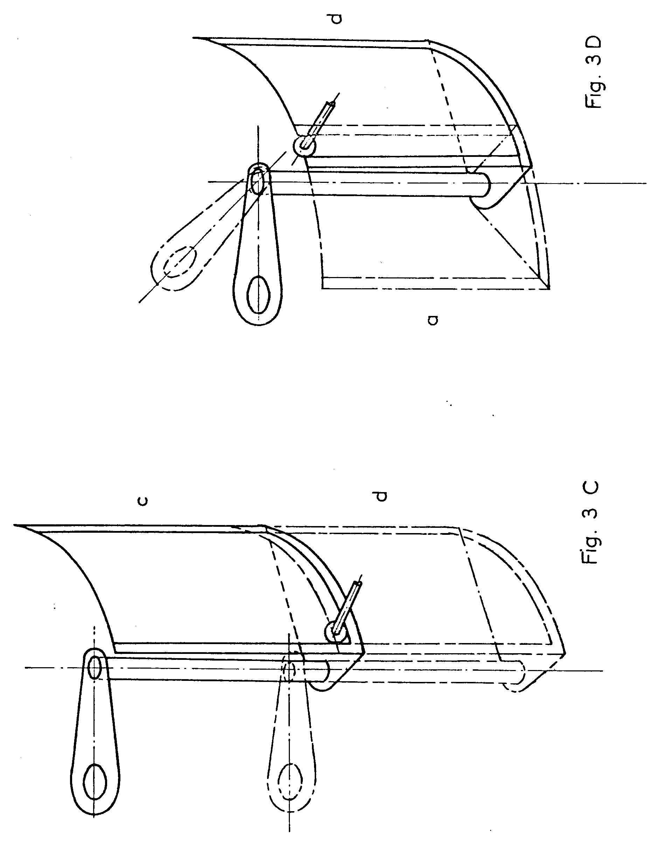

- les figures 3A, 3B, 3C et 3D montrent les mouvements successifs du moyen qui maintient et porte les lentilles de contact,
- la figure 4A et la figure 4B montrent comment le guidage, qui assure le contact entre l'organe moteur et la partie mobile de l'appareil, est effectué.
- la figure 5A et la figure 5B montrent l'aménagement de l'organe moteur pour assurer son contact avec la partie mobile et aussi pour le bloquer en fin de course.

Selon la présente invention, l'appareil stérilisateur comporte deux récipients 1 et 2 destinés à contenir respectivement la solution stérilisatrice et la solution de neutralisation, une potence 30 portant l'étui 4 renfermant les lentilles de contact.

La dite potence 30 est formée d'une traverse horizontale 3 solidaire d'un montant ou glissière verticale 5 mobile en translation suivant un axe vertical et en rotation autour du même axe, et par conséquent la partie 3 de la potence 30 qui porte l'étui 4 effectue soit un mouvement vertical suivant une parallèle au dit axe soit un mouvement de rotation suivant un arc de cercle dont le centre se trouve sur le dit axe soit un mouvement combiné de ces deux mouvements. De préférence la traverse 3 de la potence est solidaire de l'extrémité supérieure de la glissière 5.

La traverse 3 porte un couvercle 6 adapté aux récipients 1 et 2. L'étui 4 est fixé de manière amovible au dit couvercle 6 de telle sorte qu'il sera contenu dans le récipient une fois que celui-ci est couvert.

La dite potence 30 est de préférence présentée horizontalement . Un perçage prés de son extrémité libre contient le couvercle 6 en suspension.

Au départ du cycle la potence 30 est en position basse, et l'étui 4 est logé dans le récipient 1 contenant la solution stérilisatrice.

Au bout d'une durée prédéterminée fixée par un mécanisme d'horlogerie ou tout autre moyen de décompte de temps, un moyen moteur associé à ce mécanisme d'horlogerie entraîne la potence suivant un mouvement ascendant vertical, suivi par un mouvement rotatif au terme duquel l'étui 4 sera au dessus du récipient 2 et enfin suivant un mouvement descendant vertical au terme duquel l'étui 4 est immergé dans la solution de neutralisation contenue dans le récipient 2.

Le moyen moteur et le mécanisme d'horlogerie sont montés dans un boitier 7.

Un joint d'étanchéité est placé entre la glissière 5 et le boitier 7 d'horlogerie de telle sorte que le nettoyage de l'appareil de l'extérieur avec de l'eau n'expose pas à l'humidité le moyen moteur et le mécanisme d'horlogerie.

Les deux récipients 1 et 2 peuvent être fixés sur l'appareil ou pouvant être amovibles (démontables).

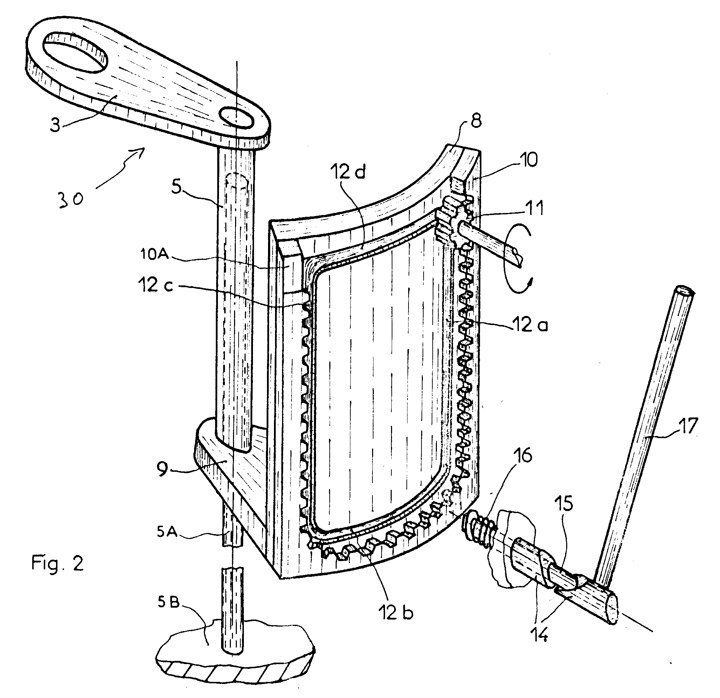

Sur la figure 2 on montre à titre d'exemple une réalisation de mécanisme qui peut donner une succession de mouvements formant un cycle : un mouvement vertical ascendant, un mouvement rotatif suivant un plan horizontal et enfin un mouvement vertical descendant.

Ce mouvement est obtenu par le mécanisme suivant :

Une platine 8 est cintrée autour de l'axe de la glissière 5 et solidaire avec celle-ci à l'aide d'un support 9.

La glissière 5 est montée sur une colonne 5a solidaire au bati 5b.

Le mouvement de la platine 8 parallèlement à ou autour de l'axe de la glissière ("l'axe de la colonne 5a") est communiqué à la potence 3 respectivement, soit sous la forme d'un même mouvement de translation, soit sous la forme d' un mouvement angulaire de même valeur que celui de la platine.

Une surépaisseur 10 est ménagée sur la surface extérieure de la platine.

Un organe rotatif 11 coopère par friction ou par obstacle avec la dite surépaisseur pour animer la platine 8 suivant l'orientation donnée à la dite surépaisseur.

Pour effectuer les trois mouvements voulus - mouvement vertical ascendant, mouvement rotatif dans un plan horizontal et mouvement vertical descendant - la surépaisseur ménagée à la surface de la platine est composée d'une partie verticale descendante suivie, à son niveau inférieur d'une partie horizontale puis d'une deuxième partie verticale montante.

Suivant une forme préférentielle de l'invention la surépaisseur 10 est une crémaillère et l'organe rotatif 11 est un pignon denté.

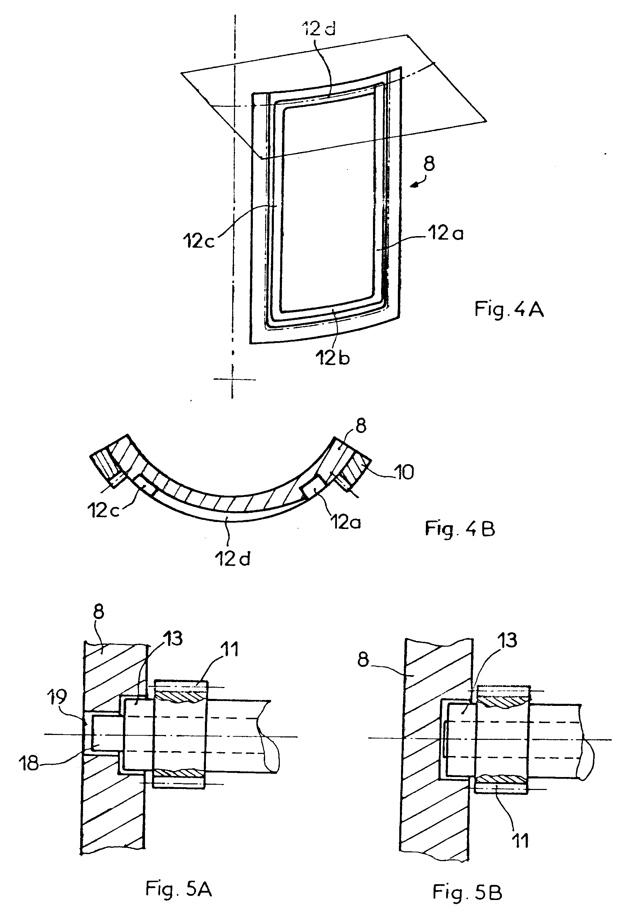

Une rainure 12 est faite parallèlement à la crémaillère sur la même face de la platine, pour loger un doigt de guidage 13 faisant saillie de l'axe du pignon dente 11 (figure 5), ceci assure le contact entre le pignon 11 et la cremaillère 8.

Cette rainure 12 est composée de quatre parties : la partie 12a parrallèle à la première partie verticale de la crémaillère pour assurer le mouvement vertical ascendant, la partie 12b parallèle à la partie horizontale de la crémaillère pour assurer le mouvement selon un plan horizontal, la partie 12c parallèle à la deuxième partie verticale de la crémaillère pour assurer le mouvement vertical descendant et la partie 12d pour assurer le passage du doigt de pignon 13 de la position finale du cycle à la position initiale de celui-ci pour permettre le commencement d'un nouveau cycle.

La profondeur de la partie 12d de la rainure a la même valeur que celle de la partie 12c au niveau de leur rencontre, par contre elle a une valeur inférieure à celle de la partie 12a au niveau de leur rencontre,comme le montre la figure (4B), qui est une vue en coupe de la figure (4A) au niveau de la partie 12d, ceci permet un passage en continiuté de 12c à 12d, mais une fois que le doigt 13 s'engage dans la partie 12a il ne peut plus retourner en 12d, ceci assure un bon contact entre la crémaillère et le pignon au commencement du cycle.

Les figures (3A), (3B), (3C) et (3D) représentent les étapes successives correspondant aux différents mouvements de la platine 8 et par conséquent de la potence 30.

Au commencement du cycle, la platine est à la position (a) comme le montre la figure (3A), l'étui de lentilles est dans le premier récipient immergé dans la solution stérilisatrice.

Au bout d'une durée prédéterminée une horloge quelconque non présentée déclenche un mécanisme qui entraîne le pignon 11 en rotation et par conséquent la platine effectue un mouvement vertical ascendant pour prendre la position (b).

A cette dernière position (b), l'étui de lentille est hors du récipient 1 mais reste toujours en regard de ce dernier.

Le pignon 11 continue à tourner, entraînant la platine jusqu'à la position c, comme le montre figure (3B). Dans cette position la partie 3 de la potence qui porte l'étui de lentilles est audessus du récipient 2. La figure (3C) montre la position (d) dans laquelle l'étui avec les lentilles sont immergés dans la solution de neutralisation contenue dans le récipient 2.

Les lentilles restent dans ce dernier en attendant leur utilisation.

La dernière partie 10a de la crémaillère 10 correspondant à la fin du cycle n'est pas dentée. Ceci permet au pignon 11 de tourner librement pour débrancher le ressort d'entraînement au cas d'un entraînement mécanique.

Dans le cas d'utilisation d'un moteur électrique, celui-ci sera autorisé à fonctionner pendant une période prédéterminée supérieure à celle nécessaire au transfert de l'étui d'un récipient à l'autre. La dite période, pendant laquelle le moteur est autorisé à fonctionner, prend en considération l'usure des piles d'alimentation et par conséquent l'allongement de la période de transfert. Pour commencer un autre cycle comme celui qu'on vient de décrire, on ramène la platine manuellement en actionnant la potence, à la position (a) .

Par sécurité et pour être sûr que l'horloge et le mécanisme d'actionnement du pignon soient mis en marche, le changement de position de la platine de la portion (d) à la position (a) sera autorisé seulement si la dite mise en marche est effectuée.

Le but pour lequel ce changement de position est conditionné par la mise en marche de l'horloge et du mécanisme d'actionnement du pignon est d'assurer le transfert de lentilles de la solution stérilisatrice à la solution de neutralisation au bout de le durée prédéterminée.
Pour assurer, selon l'invention, l'application de telles conditions, deux réalisations sont données à titre d'exemple.
Une de ces réalisations est présentée sur la figure 2, elle est constituée par une came 14 formée de deux parties, une partie fixe à travers laquelle passe un axe 15 et la deuxième partie lui est solidaire.

Un ressort 16 pousse l'axe 15 vers la platine 8. Une fois que la platine cède la place pour effectuer ses mouvements, le dit axe avance et dépasse le niveau de le platine.

Dans la position (d) de la platine, le dit axe dépasse le niveau de la platine à gauche et l'empêche de reprendre la position de départ (a).

Un levier 17 est solidaire avec l'axe 15. L'actionnement de ce levier fait tourner le dit axe et par conséquent l'éloigne de la platine. Un tel actionnement du levier peut être effectué par la même action que la mise en marche de l'horloge par exemple à l'aide du mouvement mécanique à faire pour remonter l'horloge.

Le verrouillage de la platine en fin de cycle (d) peut être encore effectué comme c'est présenté sur la figure 5. Un doigt rétractable 18 coaxial avec le pignon 11 loge, en fin de course, dans un creux 19 fait dans la platine 8. Ce doigt se retire, d'une façon classique, soit après qu'on ait remonté l'horloge, soit à l'aide d'un mécanisme quelconque pour libérer la platine.

Le fonctionnement de l'appareil est le suivant :
- On place les lentilles de contact dans leur étui,
- On place, après avoir mis l'horloge en marche, l'étui porté par la potence, dans le premier récipient contenant la solution stérilisatrice,
- Au bout d'une durée prédéterminée, la potence effectue une série de trois mouvements:

un mouvement vertical ascendant, un mouvement horizontal suivi d'un mouvement vertical descendant à la fin duquel l'étui des lentilles sera immergé dans le deuxième récipient.
- Dans cette dernière position, la platine sera bloquée et le retour à la position de départ est seulement autorisé dans le cas où la mise en marche de l'horloge est effectuée.

Il est bien évident que la succession de mouvements de la potence décrite ci-dessus peut être effectuée avec d'autres moyens purement mécaniques, hydrauliques, électromécaniques ou d'autres moyens équivalents sans sortir du cadre de la présente invention.

**Revendications**

1.  Appareil stérilisateur d'objets, par exemple des lentilles de contact, comportant un bâti (5B), deux contenants (1) et (2) montés en position fixe sur le bâti et destinés à être emplis pour l'un d'une solution stérilisatrice et pour l'autre d'une solution de neutralisation, un moyen mobile supportant un étui (4) destiné à stocker les lentilles de contact, des moyens d'entrainement du moyen mobile pour transférer l'étui (4) de stockage des lentilles de contact de la solution stérilisatrice vers la solution de neutralisation, et un mécanisme d'horlogerie déclenchant la mise en marche des moyens d'entrainement, appareil caractérisé en ce que le moyen mobile est constitué d'une potence (30) formée d'une traverse horizontale (3) et d'un montant vertical (5), ladite potence étant mobile en translation et en rotation par rapport à l'axe longitudinal du dit montant, en ce que l'étui (4) destiné à stocker les lentilles est monté sous la traverse (3) horizontale de la potence (30), en ce que les deux contenants (1) et (2) sont disposés suivant un arc de cercle centré sur l'axe de rotation de la potence en sorte de recevoir l'un après l'autre l'étui (4) des lentilles porté par la traverse (3) horizontale, et en ce que les moyens d'entrainement sont conçus pour entrainer la traverse (3) de la potence, successivement, suivant un mouvement de translation ascendant, un mouvement de rotation horizontal, et un mouvement de translation descendant, centrés sur l'axe du montant de la dite potence.

2.  Appareil selon la revendication 1 caractérisé en ce que le montant vertical de la potence est constitué par une glissière (5) tubulaire et en ce que le bâti (5B) comporte une colonne (5A) verticale sur laquelle s'engage la glissière tubulaire (5) et définissant l'axe de translation et de rotation de la potence.

**3.** Appareil selon la revendication 1 caractérisé en ce que les moyens d'entrainement de la potence sont constitués par une platine verticale (8) en forme de portion de paroi cylindrique centrée sur l'axe du montant de la potence et solidaire du dit montant par un support (9) à l'opposé de la traverse (3), et par un organe rotatif (11) coopérant en entrainement avec une surépaisseur (10) ménagée sur la platine verticale (8).

**4.** Appareil selon la revendication 3 caractérisé en ce que la dite surépaisseur (10) est en forme de U.

**5.** Appareil selon la revendication 3 caractérisé en ce que la dite surépaisseur (10) est une crémaillère et en ce que le dit organe rotatif (11) est un pignon.

**6.** Appareil selon la revendication 5 caractérisé par une rainure (12) faite parallèlement à la surépaisseur (10), dans laquelle se loge un doigt de guidage (13) lié à l'organe rotatif (11) pour assurer le contact de ce dernier avec la surépaisseur.

**7.** Appareil selon la revendication 6 dans lequel la rainure (12) est parallèle à la surépaisseur (10) sur toute sa longueur depuis la position initiale de l'organe rotatif, relativement à la dite surépaisseur, jusqu'à sa position finale, caractérisé en ce que la dite rainure (12) continue à partir de cette position finale jusqu'à la dite position initiale, formant ainsi un circuit sans fin pour permettre à l'organe rotatif de reprendre, en fin de cycle sa position initiale par rapport à la dite surépaisseur.

**8.** Appareil selon la revendication 7 caractérisé en ce que la partie (12d) de la rainure, reliant la position finale de l'organe rotatif à sa position initiale, c'est-à-dire, reliant la fin du cycle à son début, a une profondeur inférieure à celle de la rainure au début du cycle au niveau de leur rencontre.

**9.** Appareil selon l'une des quelconques revendications précédentes caractérisé par un moyen de verrouillage, par exemple un axe (15) à came ou un doigt rétractable (18), qui bloque la platine (8) en fin de cycle et permet sa mise en position initiale seulement dans le cas de la mise en marche du mécanisme d'horlogerie.

**10.** Appareil selon l'une quelconque des revendications précédentes caractérisé par un moyen assurant l'étanchéité du boîtier (7) renfermant le mécanisme au niveau du passage de la glissière (5).

**11.** Appareil selon la revendication 5 caractérisé en ce que la partie (10a) de la crémaillère correspondant à la fin du cycle de transfert, n'est pas dentée pour permettre au pignon (11) de tourner librement à la fin du dit cycle.

## Claims

**1.** Sterilizing apparatus for objects such as contact lenses, for example. The apparatus comprises a frame (5B), two containers (1) and (2) mounted on the frame in fixed position one of which serves as a recipient for a sterilizing solution, the other for a neutralizing solution, a mobile device supporting a case (4) designed to hold the contact lenses, a drive system for said mobile device to transfer the case (4) holding the contact lenses from the sterilizing solution to the neutralizing solution, and a clockwork mechanism used to control the drive mechanism. Sterilizing apparatus wherein the mobile part consists of a bracket (30) formed by a horizontal arm (3) and a vertical column (5), said bracket being capable of a translational movement and a rotary movement with respect to the vertical axis of said column, wherein the case (4) serving to hold the contact lenses is mounted under the horizontal arm (3) of the bracket (30), wherein the two containers (1) and (2) are arranged along an arc of a circle whose center is the rotation axis of the bracket in such as way as to alternately receive the case (4) holding the contact lenses carried by the horizontal arm (3), wherein the drive mechanisms are designed to successively move the arm (3) of the bracket in an ascending translational motion, a horizontal rotary motion, and a descending translational motion centered around the axis of the vertical column forming the upright of the bracket.

**2.** Apparatus as specified in claim 1 wherein the vertical column of the bracket consists of a sliding tube (5) and the frame (5B) comprises a vertical column (5A) which inserts into the sliding tube (5) and forms the translation and rotation axis of the bracket.

**3.** Apparatus as specified in claim 1 wherein the bracket drive system consists of a vertical plate (8) in the shape of a portion of a cylindrical surface centered on the axis of the bracket column and joined to the latter by a support (9) on the opposite side to the cross arm (3) and by a rotary component (11) driving a toothed overlay (10) on the vertical plate (8).

**4.** Apparatus as specified in claim 3 wherein said toothed overlay (10) is U-shaped.

5. Apparatus as specified in claim 3 wherein said overlay (10) is a toothed rack and said rotary component (11) is a gear.

6. Apparatus as specified in claim 5 wherein a groove (12) runs parallel to the overlay (10) and houses a guide stud (13) fixed to the rotary gear (11) to maintain the latter in contact with the toothed overlay.

7. Apparatus as specified in claim 6 wherein the groove (12) runs parallel to the overlay (10) along its entire length from the initial position of the rotary gear in relation to the overlay, until its final position, wherein said groove (12) continues from this final position to the initial position, thus forming a continuous circuit to allow the rotary gear to return to its initial position with respect to the overlay at the end of the cycle.

8. Apparatus specified as per claim 7 wherein the part of the groove (12d) joining the final position of the rotary gear to its initial position, i.e. linking the end of the cycle to the beginning, is shallower then the groove at the beginning of the cycle, where the two portions meet.

8. Apparatus as specified in any of the preceding claims wherein a locking mechanism, for instance a spindle and cam (15) or a retractable stud (18) which locks the plate (8) at the end of the cycle and allows for transition to its initial position only when the clockwork mechanism is operating.

10. Apparatus as specified in any of the preceding claims wherein an element seals the unit casing (7) enclosing the mechanism at the place where the sliding tube (5) passes through.

11. Apparatus as specified in claim 5 wherein the part (10a) of the rack corresponding to the end of the transfer cycle is not toothed, so that the gear (11) is able to rotate freely at the end of said cycle.

## Patentansprüche

1. Sterilisierungsgerät zum Sterilisieren bestimmter Objekte, zum Beispiel Kontaktlinsen, das aus einem Gestell (5B), zwei Behältern (1) und (2), welche in fester Stellung auf dem Gestell angebracht sind und dazu dienen, mit einer sterilisierenden bzw. einer neutralisierenden Lösung gefüllt zu werden, aus einer mobilen Vorrichtung, welche ein zum Aufbewahren der Kontaktlinsen dienendes Etui (4) trägt, aus Antriebsvorrichtungen für die mobile Vorrichtung, um das Aufbewahrungsetui (4) der Kontaktlinsen von der sterilisierenden zur neutralisierenden Lösung zu befördern, sowie einem das Ingangsetzen der Antriebsvorrichtungen auslösenden Uhrwerk, besteht. Dieses Gerät kennzeichnet sich dadurch, daß die mobile Vorrichtung aus einem mittels eines horizontalen Querbalkens (3) und eines vertikalen Ständers gebildeten Träger (30) besteht (5), wobei dieser Träger bezüglich der Längsachse des besagten Ständers per Translation bzw. Rotation beweglich ist. Ein weiteres Kennzeichen ist, daß das zum Aufbewahren der Kontaktlinsen dienende Etui unter dem horizontalen Querbalken (3) des Trägers (30) angebracht ist, daß die beiden Behälter (1) und (2) so angeordnet sind, daß sie sich, einen Kreisbogen bildend, um die Rotationsachse des Trägers drehen, um so nacheinander das von dem horizontalen Querbalken (3) herangeführte Kontaktlinsenetui in Empfang nehmen zu können (4), und daß die Antriebsvorrichtungen, um den Querbalken (3) des Trägers anzutreiben, so konzipiert sind, daß sie nacheinander eine aufwärts gerichtete Translations-, eine horizontale Rotations- und eine abwärts gerichtete Translationsbewegung ausführen, wobei sie sich um die Ständerachse des besagten Trägers drehen.

2. Das wie unter Patentanspruch 1 beschriebene Gerät kennzeichnet sich dadurch aus, daß der vertikale Ständer des Trägers aus einer röhrenförmigen Laufschiene (5) besteht und dadurch, daß sich auf dem Gestell (5B) eine vertikale Säule (5A) befindet, in die sich die Laufschiene einfügt und die die Translations- und Rotationsachse des Trägers bildet.

3. Das wie unter Patentanspruch 1 beschriebene Gerät kennzeichnet sich dadurch aus, daß die Antriebsvorrichtungen des Trägers aus einer vertikalen Platte (8) bestehen, welche die gleiche Form hat wie ein Teil aus einer zylinderförmigen Wand, sich um die Ständerachse des Trägers dreht und mittels einer dem Querbalken (3) gegenüberliegenden Auflage (9) sowie eines rotierenden Organs (11) aus einem Stück mit dem besagten Ständer besteht, wobei das rotierende Organ beim Antrieb mit einer auf der vertikalen Platte (8) ausgesparten Überdikke (10) zusammenwirkt.

4. Das wie unter Patentanspruch 3 beschriebene Gerät kennzeichnet sich dadurch aus, daß die besagte Überdicke U-förmig ist.

5. Das wie unter Patentanspruch 3 beschriebene Gerät kennzeichnet sich dadurch aus, daß die besagte Überdicke (10) eine Zahnstange ist und das besagte rotierende Organ (11) ein Ritzel.

6. Das wie unter Patentanspruch 5 beschriebene Gerät kennzeichnet sich durch eine parallel zur Überdicke (10) angebrachte Nut (12) aus, in die sich ein Führungsstift (13) einfügt, welcher mit dem rotierenden Organ (11) verbunden ist, um so den Kontakt zwischen letzterem und der Überdicke zu gewährleisten.

7. Das wie unter Patentanspruch 6 beschriebene Gerät, bei dem die Nut (12) parallel zur Überdicke ist, und zwar in ihrer gesamten Länge von der Ausgangsposition des rotierenden Organs an, im Verhältnis zur besagten Überdikke, bis zu seiner Endposition, kennzeichnet sich dadurch aus, daß die besagte Nut (12) von dieser Endposition an bis hin zur besagten Ausgangsposition weiterläuft und so ein endloser Kreislauf entsteht, aufgrund dessen das rotierende Organ am Ende eines Zyklusses bezüglich der besagten Überdicke wieder in die Ausgangsposition zurückgehen kann.

8. Das wie unter Patentanspruch 7 beschriebene Gerät kennzeichnet sich dadurch aus, daß der Teil der Nut, der die Endposition des rotierenden Organs mit der Ausgangsposition verbindet, das heißt, das Ende eines Zyklusses mit dem Anfang verbindet, eine Tiefe hat, die niedriger ist als die der Nut zu Beginn des Zyklusses bei deren Aufeinandertreffen.

9. Das wie unter einem beliebigen der obengenannten Patentansprüchen beschriebene Gerät kennzeichnet sich durch eine Verriegelungsvorrichtung aus, z.B. einem Nockenbolzen oder einem einschiebbaren Zapfen, welche die Platte (8) am Ende eines Zyklusses blockiert und so deren Zurückgehen in die Ausgangsposition nur dann ermöglicht, wenn das Uhrwerk angeht.

10. Das wie unter einem beliebigen der obengenannten Patentansprüchen beschriebene Gerät kennzeichnet sich dadurch aus, daß eine Vorrichtung für die Dichtheit des Gehäuses sorgt, welches den Mechanismus (7) auf der Höhe der Laufschiene (5) einschließt.

11. Das wie unter Patentanspruch 5 beschriebene Gerät kennzeichnet sich dadurch aus, daß der Abschnitt (10a) der Zahnstange, der dem Ende des Übertragungszyklusses entspricht, nicht gezahnt ist, damit das Ritzel am Ende des besagten Zyklusses frei rotieren kann.

Fig.1

Fig. 2

Fig. 3B

Fig. 3A

Fig. 3 D

Fig. 3 C

Fig. 4 A

Fig. 4 B

Fig. 5A

Fig. 5B